(19) Europäisches Patentamt / European Patent Office / Office européen des brevets

(11) **EP 3 568 131 B1**

(12) **EUROPEAN PATENT SPECIFICATION**

(45) Date of publication and mention of the grant of the patent:
**18.11.2020 Bulletin 2020/47**

(21) Application number: **18703360.0**

(22) Date of filing: **11.01.2018**

(51) Int Cl.:
*A61K 31/365* (2006.01)        *A61P 17/04* (2006.01)
*A61P 17/06* (2006.01)        *A61P 17/14* (2006.01)

(86) International application number:
**PCT/IB2018/050170**

(87) International publication number:
**WO 2018/130959 (19.07.2018 Gazette 2018/29)**

(54) **COMPOSITION COMPRISING 7-HYDROXYMATAIRESINOL FOR USE IN THE TREATMENT AND IN THE PREVENTION OF TRICHOLOGICAL DISEASES OF INFLAMMATORY AND/OR HORMONAL ORIGIN**

ZUSAMMESETZUNG ENTHALTEND HYDROXYMATAIRESINOL ZUR BEHANDLUNG UND PRÄVENTION VON TRICHOLOGISCHEN ERKRANKUNGEN ENTZÜNDLICHEN UND/ ODER HORMONALEN URSPRUNGS

COMPOSITION COMPRENANT DU 7-HYDROXYMATAIRESINOL POUR LE TRAITEMENT OU LA PREVENTION DES MALADIES TRICHOLOGIQUES DE L'ORIGINE INFLAMMATOIRE OU HORMONELLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.01.2017  IT 201700002306**

(43) Date of publication of application:
**20.11.2019  Bulletin 2019/47**

(73) Proprietors:
• **Sinerga Group S.r.l.**
  **20154 Milano (IT)**
• **Linnea S.A.**
  **6595 Riazzino (Locarno) (CH)**

(72) Inventors:
• **FONTANA, Marco**
  **20154 Milano (IT)**
• **PACCHETTI, Barbara**
  **CH-6595 Riazzino - Ticino (CH)**

(74) Representative: **Perani & Partners S.p.A.**
**Piazza Armando Diaz, 7**
**20123 Milano (IT)**

(56) References cited:
**WO-A1-2004/000304      US-A1- 2003 100 514**
**US-A1- 2007 166 255**

• **Staff Reporter: "Norway spruce lignan dominan lignan in cereals, says study", , 19 July 2008 (2008-07-19), XP002780144, Retrieved from the Internet: URL:https://www.nutraingredients.com/Artic le/2006/10/26/Norway-spruce-lignan-dominan t-lignan-in-cereals-says-study [retrieved on 2018-04-17]**
• **ANNIKA I. SMEDS ET AL: "Quantification of a Broad Spectrum of Lignans in Cereals, Oilseeds, and Nuts", JOURNAL OF AGRICULTURAL AND FOOD CHEMISTRY, vol. 55, no. 4, 1 February 2007 (2007-02-01), pages 1337-1346, XP055467697, US ISSN: 0021-8561, DOI: 10.1021/jf0629134**
• **Abich, Evaluation of trans membrane diffusion of 7-Hydroxymatairesinol across Strat-M membrane contained in cosmetic raw materials**

Remarks:
The file contains technical information submitted after the application was filed and not included in this specification

EP 3 568 131 B1

**Description**

FIELD OF THE INVENTION

The subject of the present patent application is a composition comprising 7-hydroxymatairesinol for use in the treatment of diseases affecting the hair and scalp.

PRIOR ART

Lignans are vegetal compounds belonging to the class of non-flavonoid polyphenols, characterized by a diphenolic ring that makes them similar to endogenous oestrogens.

A source of purified lignans is 7-hydroxymatairesinol, isolated from the nodes of *Picea Abies.*

Lignans act like oestrogens, stimulating the synthesis and the levels in the circulation of sexual hormones. In fact, they regulate the balance of oestrogens in the body in a positive way, binding their receptors and simulating their action.

7-hydroxymatairesinol, acting on the balance of oestrogens, has shown effects on menopause, in particular on hot flashes, besides having a preventive action on cardiovascular diseases.

7-hydroxymatairesinol has interesting anti-inflammatory properties, stimulates protein synthesis and cell proliferation on fibroblasts of the dermal papilla of the hair follicle.

Moreover, due to its strong antioxidant power, it also has a beneficial effect on prostate health.

The European patent EP1513514 describes topical formulations comprising 7-hydroxymatairesinol as an active principle for cosmetic use, for the reduction of time- and photo-induced skin aging signs.

The patent further describes the use of hydroxymatairesinol as a preservative in the preparation of cosmetic formulations, to prevent the oxidation of other active components.

The international patent application WO 2015/173345 describes a composition comprising 7-hydroxymatairesinol for the improvement of the metabolic syndrome, more specifically, it refers to a pharmaceutical composition or a food product capable of preventing, relieving or treating the metabolic syndrome.

SUMMARY OF THE INVENTION

The Applicants have now developed a composition comprising 7-hydroxymatairesinol, capable of increasing the local bioavailability of the active principle, having an important anti-inflammatory action in vitro and a mitogenic action in the dermal papilla.

Said anti-inflammatory and mitogenic actions are used in the treatment of hair loss, when induced by inflammatory phenomena and hormone imbalance.

The object of the present patent application is therefore a composition for application on the hair or on the scalp, comprising

- 7-hydroxymatairesinol in concentrations between 2% and 10% by weight on the total weight of the composition,
- diols and/or triols in a concentration between 30% and 60% by weight on the total weight of the composition,
- lipoaminoacids in a concentration between 30% and 60% by weight on the total weight of the composition,

to be used in the treatment or prevention of trichological diseases of inflammatory and/or hormonal origin.

A further object of the present patent application is a formulation comprising said composition in combination with further active principles, suitable excipients and/or eluents.

DESCRIPTION OF THE FIGURES

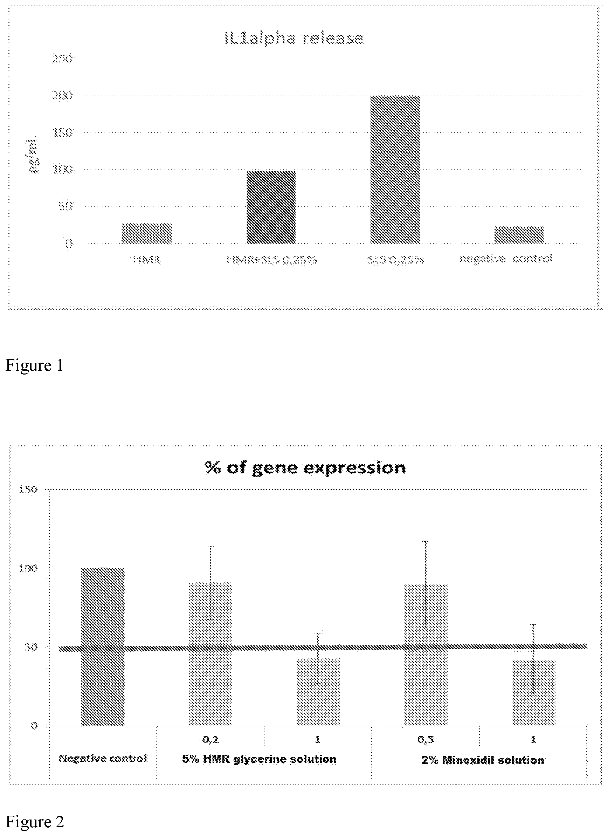

The anti-inflammatory activity evaluation test was carried out on reconstituted 3D epidermis. Figure 1 shows the percentage release of the cytokine IL1-$\alpha$ from cells in the culture medium. The respectively analysed epidermis samples are as follows:

- untreated (negative control),
- treated with sodium lauryl sulphate (SLS) at 0.25% (positive control),
- treated only with 7-hydroxymatoisoresinol (HMR) at 0.25%,
- treated with SLS at 0.25% and with HMR at 0.25%.

For the in vitro evaluation of the gene expression profile of the enzyme 5$\alpha$-reductase on human cells of the dermal papilla, please see Figure 2, which shows the changes in the gene expression profile of 5$\alpha$-reductase, respectively in relation to:

- negative control,
- 5% HMR glycerine solution (0.2 mg/ml and 1 mg/ml), respectively 0.01% HMR and 0.05% HMR,
- 2% Minoxidil solution, lotion according to Official Pharmacopoeia, XII Edition (1 mg/ml and 0.5 mg/ml).

DETAILED DESCRIPTION OF THE INVENTION

For the purposes of the present invention, the word "comprising" is to be meant as including at least all the explicitly mentioned elements, but also containing the optional, additional and not explicitly declared elements.

On the other hand, the term "consisting" is to be meant as including only the explicitly mentioned characteristics and no more than those.

Composition means the set of ingredients necessary for the delivery of an active principle, regardless of the pharmaceutical/cosmetic form in which said active principle is to be included (in the examples referred to as "carrier composition").

For the purposes of the present invention, lipoaminoacids mean fatty acid derivatives with hydrolysed proteins or single amino acids.

More preferably, lipoaminoacids are molecules deriving from a "green" technology, resulting from the reaction between fatty acids and hydrolysed plant proteins or single amino acids obtained by biofermentation.

Preferably, the fatty acid has a number of carbon atoms included between C6-C20, more preferably between C8-16.

The lipoaminoacids included in the composition according to the invention comprise fatty acids such as, e.g. caprylic, lauric and myristic acid.

More preferably, said fatty acids are coconut fatty acids.

Amino acids are preferably selected from the group of natural amino acids, such as e.g. alanine, phenylalanine, glycine, isoleucine, leucine, methionine, proline, tryptophan, valine, asparagine, glutamine, serine, tyrosine, threonine, cysteine, aspartate, glutamic acid, arginine, histidine, lysine, more specifically selected from glutamic acid, phenylalanine, isoleucine, leucine, lysine, proline, arginine, cysteine and valine.

More preferably, amino acids are selected from cysteine, proline and arginine.

The lipoaminoacids included in the composition according to the invention comprise hydrolysed plant proteins such as oats, wheat and soy.

More preferably, the hydrolysed proteins of choice are oat proteins.

Diols and triols suitable for the preparation of the composition object of the present patent application are selected, e.g. from 1,2-propanediol, butanediol, 1,3-propanediol, glycerol.

Trichology indicates the branch of medicine that studies the anatomy, physiology and pathology of hair and body hair.

Trichological pathology indicates a chronic or acute disorder affecting the scalp and the hairy areas of the epidermis.

More specifically, trichological diseases of inflammatory origin mean those pathologies characterized by an inflammation of the scalp or of the piliferous areas induced by internal factors, such as e.g. genetic predisposition, hormone imbalance, psychological or emotional fatigue, excessive workload, change of season or lack of sleep, and by external factors, such as frequent hair drying and styling with the hair dryer, pollution, UV rays.

Inflammation usually appears with symptoms such as itching and discomfort or burning in the scalp.

The combination of these internal and external stress factors determines an alteration in the cellular homeostasis of the hair bulb, with an imbalance between production and elimination of peroxides and free radicals, which are components harmful to the cell.

Under the stimulus of free radicals, the cells produce pro-inflammatory chemical agents that cause inflammation of the scalp, responsible for a weakening of the hair bulb and for a consequent hair loss.

Using molecular modelling studies, the Applicants have identified potential enzymatic targets of 7-hydroxymatairesinol for its innovative administration aiming to slow down the hair loss.

These studies showed an interesting score value of arachidonate 5-lipoxygenase, an enzyme involved in the synthesis of leukotrienes from arachidonic acid.

In fact, arachidonic acid, released by lipid membranes in response to damage and irritation, is converted by arachidonate 5-lipoxygenase into (6E, 8Z, 11Z, 14Z) - (5S) -5-hydroperoxicose-6,8,11,14-tetrahenoate, precursor of leukotrienes. These leukotrienes are, in turn, related to the production of inflammation mediators, such as histamine and prostaglandins.

Various evidence suggests, given its involvement in the inflammatory response, a key role of arachidonate 5-lipoxygenase in hair loss, for example in alopecia areata (AA).

Preferably, the composition object of the present patent application aims to the treatment and prevention of trichological diseases of inflammatory and/or hormonal origin characterized by hair loss.

Hair loss means an acute or chronic abnormal condition of hair replacement, in which the amount of falling hair is visibly greater than the amount of growing hair.

More preferably, the inflammatory trichological diseases responsible for hair loss include e.g. dandruff, asteatosis, aerated alopecia, seborrhoea, folliculitis, ringworm and tricodynia are included.

Dandruff means scalp desquamation. It can be of two types, oily or dry. In the case of oily dandruff, the scales remain attached to the hair and, if the problem persists for a long time with itching of the scalp, it can promote hair loss.

Asteatosis, or dry skin, is similar to dry dandruff, since it also is a hydrolipid problem.

Alopecia areata is an autoimmune disease mediated by T cells and tissue-specific hair follicles with a genetic predisposition to hair loss. It has an incidence of 2% on the entire population of both sexes, of all age groups and of all ethnic groups. It is characterized by irregular areas without hair or sometimes even body hair, in different areas of the body, such as eyebrows, eyelashes or beards. It has an autoimmune origin, since the immune system mistakenly attacks the hair follicles, slowing down the production of hair.

Seborrhoea is due to excessive abundance of sebum, which prevents proper breathing of the scalp. It is associated with itching, localized pain, sometimes with seborrheic alopecia.

Folliculitis refers to an inflammation of the follicles, due to excessive friction of the scalp. It is favoured by an excess of sebum. Due to the itching and the consequent habit of scratching, it can lead to hair loss.

Ringworm is caused by a fungus, produces patchy rashes, with severe itching and scalp pain.

Tricodynia indicates painful sensation on the scalp, with increased intensity when brushing or caressing the hair. It is sometimes accompanied by itching or tingling, with chronic or periodic manifestations, and is caused by factors that are usually harmless or unrecognizable (allodynia). Tricodynia seems to be associated with stress and anxiety.

Trichological diseases of hormonal origin are diseases induced by the action of androgens on the hair bulb in subjects genetically predisposed to have a greater sensitivity to the biological effect of said hormones.

Even more preferably, trichological pathology of hormonal origin means androgenetic alopecia.

Androgenetic alopecia is a disorder characterized by the slow and progressive miniaturization of the hair follicles on the scalp and therefore of the hair produced by them. Known in the common language as baldness, it is the most widespread type of hair loss that affects about 80% of men and 50% of women.

The miniaturization of the hair bulb, characteristic of this disorder, occurs as a result of the transformation of testosterone into its most active metabolite, dihydrotestosterone (DHT) by the enzyme $5\alpha$-reductase, expressed primarily in the prostate, testicles, cells of the hair follicle and of the adrenal glands. The miniaturization leads to the definitive death of the bulb and to the irreversible loss of the hair.

Given the pathologies to which the composition object of the present invention is addressed, the mitogenic and anti-inflammatory activity exhibited by 7-hydroxymatairesinol has been experimentally demonstrated by the Applicants through in vitro studies on cell cultures of dermal papilla of human hair follicle and three-dimensional systems of reconstituted human epidermis.

7-hydroxymatairesinol also exhibits inhibitory activity against $5\alpha$-reductase, similar to the one carried out by Minoxidil, which is currently considered the drug mainly used in trichological anti-hair fall and reinforcing treatments.

Please refer to the experimental section of the present application for a more exhaustive explanation of the activity exhibited by 7-hydroxymatairesinol.

According to a preferred embodiment, 7-hydroxymatairesinol is contained in the composition in question in amounts ranging from 3% to 6% by weight, based on the total weight of the composition.

Preferably, the composition according to the present invention comprises diols and/or triols in concentrations ranging from 35% to 50% by weight based on the total weight of the composition.

Preferably, the composition according to the present invention comprises lipoaminoacids in a concentration ranging from 45% to 60% by weight based on the total weight of the composition.

According to a preferred embodiment, the formulation comprises the composition in amounts ranging from 4% to 8% by weight based on the total weight of the formulation.

A further object of the present patent application is a formulation comprising the composition for use, according to the present invention, in combination with possible further active principles and suitable eluents and/or excipients.

Active principles capable of increasing the microcirculation and with vasodilatory properties, such as Xsolve (*Ethyl Ximenynate, Lecithin*) are among the additional active principles that may be possibly included in the formulation object of the present application,

An improvement in the local microcirculation could in fact further promote the HMR permeation and absorption, thus making it more effective on the hair bulb.

The formulation according to the present invention may possibly contain 7-hydroxymatairesinol as the only active principle.

Preferably, the formulation object of the present application is prepared in the form of liquid, fluid, or semi-fluid or consistent formulations.

Said formulations are, for example, selected among shampoos (category: foaming products), leave on (no-rinse) anti-hair loss hydrogels and lotions, rinse off (with rinsing) conditioners and cationic masks, mousses.

The distribution of 7-hydroxymatairesinol in lipoaminoacids entails the advantage of a complete solubilisation of the active principle and, consequently, an increase in its local bioavailability, thus improving its effectiveness on the scalp and the hair.

Diols and/or triols constitute the carrier wherein HMR and lipoaminoacids are solubilized, advantageously favouring the formation of the vehiculation composition of the active principle.

Lipoaminoacids mimic the physiological barrier of the skin, thus preserving the hydro-lipid balance essential for the protection and maintenance of the skin characteristics, such as elasticity and hydration. They are stable, easy to use, have considerable sensory properties and are strongly dermo-like and able to convey more effectively the active principle.

**Experimental Section**

1. ASSESSMENT OF THE MITOGEN ACTIVITY OF 7-**HYDROXYMATAIRESINOL ON DERMAL PAPILLA FIBROBLASTS**

1.1 Performed tests

- Cell viability test after cell cycle synchronization with dermal papilla cells of the human hair follicle cultured in adhesion for the evaluation of the cell proliferation potential induced by the tested sample.
- Evaluation test of total protein content in human fibroblasts of the hair dermal papilla exposed to the sample in question using scalar concentrations and different treatment times.

1.2 Experimental Protocol

1.2.1 Purpose of the test

The purpose of the test is quantitatively establishing the effects of 7-hydroxymatairesinol (HMR) on cell proliferation and on the synthesis of total proteins in human fibroblasts of the dermal papilla through the cell viability test (MTT test) after cycle synchronization, and establishing the protein dosage at different exposure times and at different sub-toxic sample dilutions.

The dermal papilla of the hair is a structure of primary importance for the hair follicle, whose function is feeding the piliferous root. It is mainly constituted by fibroblasts, whose main function is producing collagen and elastin, fundamental protein-based components of hair fibres. If a substance/compound is able to determine an increase in cell proliferation and protein synthesis, this suggests its role in stimulating follicle growth and entry into anagenesis.

1.2.2 Cell Model

Dermal papilla cells derived from human hair follicle (HHDPC: human hair dermal papilla cells) with fibroblastic morphology. The cells are cultured in DMEM containing 10% FBS and antibiotics.

1.2.3 Treatment and Exposure

The cells were seeded in 24-well plates and left to grow for 24h at 37°C and 5% CO2. Then they were deprived of the serum 6 hours before the experiment for cell cycle synchronization.

After 6 hours, fresh culture medium was added without serum but containing the sample to be tested in order to reach different final dilutions. The 5% glycerine-solubilized sample was dissolved in the culture medium. The test was carried out in triplicate.

After 24 and 48 hours of exposure, the MTT cell viability test and the total protein test using the Bradford method[1] were carried out on separate plates. Untreated cells were used as a negative control (CN), while cells treated with human insulin were used as a positive control (CP).

1.2.4 MTT cell viability test

This method was originally developed by Mossman[2]. After 24 and 48 hours of treatment, cell viability was evaluated by incubating the cells with MTT (3-[4,5-dimethylthiazol-2-yl]-2,5-diphenyl tetrazolium bromide) for 2 hours.

The precipitated formazan crystals were then extracted using isopropanol or DMSO and were spectrophotometrically quantified at 570 nm against 650 nm. The absorbance was read with a colorimeter (Tecan, model Sunrise remote) equipped with a plate reader subtracting the reading of the bottom.

1.2.5 Total protein dosage

The protein dosage was carried out according to the Bradford method. At the end of the treatment, the cells were washed with sterile PBS and lysed by treatment with purified water at 4°C. The colouring reagent was added to the lysate. A standard curve of albumin titrated to 20, 40, 80 and 100 $\mu$g/ml was prepared in a similar manner.

Then 200 $\mu$l of each sample, controls and standards, each in triplicate, were transferred to a 96-well plate. The reading was carried out with a colorimeter (Tecan, Sunrise) equipped with a plate reader, at 595 nm.

1.3 Result expression

The cell viability was expressed as a percentage according to the formula:

$$\% \text{ of cell viability} = [\text{OD (570 nm - 650nm) tested product/OD (570 nm - 650 nm) CN}] \times 100$$

OD: Optical density

To quantify proteins, a standard albumin calibration curve was drawn and sample concentrations were calculated using the interpolation curve formula and relative absorbances. The averages of the individual data were then calculated, and for each sample the percentage of increase in protein synthesis was calculated, with respect to the negative control:

$$\% \text{ protein synthesis increment} = (\mu\text{g/ml sample protein}/\mu\text{g/ml CN})*100$$

1.4 Method acceptability criteria

| | |
|---|---|
| for the negative control (CN): | the standard deviation must be ≤ 18% |
| for the positive control (CP): | the increase in protein synthesis and cell proliferation must be > 10% if compared to the negative control, the standard deviation must be ≤ 18% |
| for the sample: | the standard deviation must be ≤ 18% |

1.5 Results and conclusions

1.5.1 Test acceptability requirements

Table 1

| | Value | | Limits | Result |
|---|---|---|---|---|
| **CN** % standard deviation | ≤ 18% | | ≤ 18 | Complies |
| **CP** % increase | >10 % MTT | >10% proteins | >10% | Complies |
| **CP** % standard deviation | ≤ 18 | | ≤ 18 | Complies |

(continued)

|  | Value | Limits | Result |
|---|---|---|---|
| **Sample** % standard deviation | ≤ 18 | ≤ 18 | Complies |

1.5.2 Cell growth test

Table 2

| HMR (5% glycerine) Batch: n.p. | % cell viability | |
|---|---|---|
|  | 24 h | 48 h |
| 1 mg/ml | 99.66 | 104.73 |
| 0.2 mg/ml | *110.41* | *119.01* |
| Human insulin 100 μg/ml | *129.72* | *145.85* |

1.5.3 Total protein dosage

Table 3

| HMR (5% glycerine) Batch: n.p. | % protein synthesis | |
|---|---|---|
|  | 24 h | 48 h |
| 1 mg/ml | *24.46* | *16.76* |
| 0.2 mg/ml | *10.36* | -------- |
| Human insulin 100 μg/ml | *65.20* | *62.31* |

1.5.4 Conclusions

Based on the above results, 7-hydroxymatairesinol is able to stimulate protein synthesis (+ 24%) and cell proliferation (+ 19%) on fibroblasts of human dermal papilla if compared to untreated cells.

This is a sign of a potential stimulation of hair growth and strengthening.

## 2. IN VITRO EVALUATION OF THE ANTI-INFLAMMATORY POTENTIAL OF 7-HYDROXYMATAIRESINOL ON RECONSTITUTED HUMAN EPIDERMIS

2.1 Test

- Cytotoxicity through the MTT test at the end of incubation: cell survival test with 3D human epidermis reconstituted in vitro for the evaluation of the cytotoxic potential caused by the sample.

- Inhibition of the release of cellular mediators of inflammation (IL-1$\alpha$) after stress induced with SLS.

2.2 Experimental protocol

2.2.1 Purpose of the test

The study concerns the in vitro evaluation of the anti-inflammatory potential of 7-hydroxymatairesinol against the inflammatory reaction induced by moderate irritants such as sodium lauryl sulphate (SLS), using a model of human skin cells grown in vitro in multilayer.

The inhibition of the cytokine IL-1$\alpha$ release caused by SLS is investigated by specific ELISA tests in the presence of the tested substance compared to untreated skin samples.

2.2.2 Cell model

The cell model used for in vitro tests is represented by a three-dimensional system of reconstituted human epidermis in vitro.
This three-dimensional cellular model is made up of normal human primary keratinocytes, left to grow in vitro in order to simulate the multi-layered architecture of the human epidermis, with a well-differentiated stratum corneum. The model was purchased from CELLSYSTEMS (Troisdorf, batch 100-AE1818-1).

2.2.3 Treatment and exposure

The skin irritation characterized by the release of IL-1$\alpha$ was caused by an epidermis pre-treatment for 60 minutes with SLS 0.25%.
Subsequently, 30$\mu$l of diluted sample were placed on two units and the exposure was extended for 24 hours at 37°C, 5% CO2. The sample was diluted to 0.25% in phosphate buffer.
As a negative control, two units of epidermis were treated with phosphate buffer (PBS), as a positive control two units were treated for 60' with 0.25% SLS. The test was carried out in duplicate.
At the end of the exposure, the sample and the references were removed by washing with phosphate buffer (PBS) and the cell viability was assessed.
For the analysis of IL-1$\alpha$, samples of culture medium were taken at 6 and 24 hours. The maintenance medium of the units treated with PBS and the medium of two non-irritated skin units treated only with the sample were taken as negative control, whereas the culture medium of two skin units treated with the 0.25% SLS solution was used as a positive control.

2.2.4 MTT cell viability test

The epidermis was incubated for 3h at 37°C with a lmg/ml solution of MTT (3- [4,5-dimethylthiazol-2-yl] -2,5-diphenyl tetrazolium bromide).
Then the solution was removed and replaced with isopropanol with subsequent 2 h incubation at room temperature.
Two aliquots of each sample were transferred to a 96-well plate for reading the absorbance, which was carried out at the wavelength of 570 nm, with a colorimeter (Tecan, model Sunrise remote) equipped with a plate reader.

2.2.5 IL-1$\alpha$ analysis

In this study, IL-1$\alpha$ was dosed using the direct-type ELISA (Enzyme-linked immunosorbent assay) method, i.e. the colour development was directly proportional to the amount of cytokines in the culture medium.
The reading was carried out at 450 nm. The sensitivity limit of this test was less than 10 pg/ml.

2.3 Result expression

The result was expressed as a cellular viability in percent according to the formula: % cell viability = [OD (570 nm) tested sample/OD (570 nm) CN] x 100
The IL-1$\alpha$ concentration was determined using a standard curve as a reference. The inhibition of IL1-$\alpha$ release was determined as:
% IL-1$\alpha$ inhibition: 100- (pg/ml IL-1$\alpha$ released from the irritated sample/pg/ml IL-1$\alpha$ positive control)* 100

2.4 Method acceptability criteria

| | |
|---|---|
| for negative control (CN): | OD between 1.0-3.0 the standard deviation must be $\leq$ 18% |
| for the positive control (CP): | the average viability value (expressed as % with respect to the CN) must be < 70%, the standard deviation must be $\leq$ 18% |
| for the sample: | the standard deviation must be $\leq$ 18% |

## 2.5 Results and conclusions

### 2.5.1 Test acceptability requirements

Table 4

| MTT | | Value | Limits | Result |
|---|---|---|---|---|
| Positive control | Average viability | 50.05% | <70% | Complies |
| | % standard deviation | 1.11 | $\leq$ 18% | Complies |
| Negative control | OD | 1.82 | 1.0-3.0 | Complies |
| | % standard deviation | 4.83% | $\leq$ 18% | Complies |
| Sample: | % standard deviation | 3.74 | $\leq$ 18 | Complies |

### 2.5.2 Results

Table 5

| MTT | % average cell viability (ds) |
|---|---|
| HMR | 78.23 (3.74) |
| HMR + SLS 0.25% | 55.92 (1.31) |
| SLS 0.25% Positive Control (CP) | 50.05 (1.11) |
| Negative control (PBS) | 100.00 (4.83) |

Table 6

| | IL-1$\alpha$ pg/ml | | | |
|---|---|---|---|---|
| | 6 h | | 24 h | |
| | Mean pg/ml | % inhibition | Mean pg/ml | % inhibition |
| HMR | 18.07 | | 26.40 | |
| HMR + SLS 0.25% | 19.72 | *13.94* | 97.70 | *51.32* |
| SLS 0.25% Positive Control (CP) | 22.92 | | 200.72 | |
| Negative control (PBS) | 4.79 | | 23.12 | |

See also Figure 1 for the quantification in pg/ml of the release of Interleukin 1$\alpha$ (IL-1$\alpha$) released in each of the two samples treated with HMR, HMR + SLS 0.25%, of the positive control treated with SLS 0.25 % and of the negative control treated with PBS.

## 2.6 Conclusions

Based on the reported results, 7-hydroxymatairesinol has an anti-inflammatory activity in vitro.

In fact, after 24 hours of incubation there is a reduction in the release of IL-1$\alpha$ of 51.32%.

**3. IN VITRO EVALUATION OF THE GENE EXPRESSION PROFILE OF THE 5-REDUCTASE ENZYME ON HUMAN CELLS OF THE DERMAL PAPILLA**

3.1 Test

Measurement of the gene expression level of $5\alpha$-reductase by means of Real Time Polymerase Chain Reaction (qRT-PCR) in human cells of the dermal papilla with and without treatment with the sample.

3.2 Purpose of the test

The purpose of the test is to highlight and quantify changes in gene expression of $5\alpha$-reductase levels in cell cultures exposed to treatment with the tested substance compared to controls of the same untreated cells. This enzyme is expressed in skin melanocytes, fibroblasts and keratinocytes and turns testosterone into di-hydro-testosterone, a key metabolite implicated in various skin disorders such as acne vulgaris, hirsutism, seborrhoea and androgenetic alopecia.

After birth, the $5\alpha$-reductase enzyme (type 1) is expressed in multiple body areas, including liver, skin, scalp and prostate. $5\alpha$-reductase (type 2) is expressed in prostate, seminal vesicles, epididymis, liver, and, to a lesser extent, scalp and skin. In this test, the cells are previously treated with testosterone to induce $5\alpha$-reductase production and are exposed to various test substances for 48 hours. A titrated extract of Serenoa repens, a known pharmacologically active compound used to treat benign prostatic hyperplasia, is used as a positive control. The analysis is carried out through RT-qPCR (Real Time Quantitative Polymerase Chain Reaction), a very sensitive method for the detection and quantification of messenger RNA coding for the protein in analysis and therefore for the corresponding gene activation. RT-qPCR measures the fluorescence emitted by the amplicons produced during each PCR cycle.

3.3 Test execution:

3.3.1 Cell model:

A culture of human dermal papilla (HHDPC) cells was used. The cells were grown in MEM containing 10% FBS and antibiotics.

3.3.2 Treatment and Exposure:

The cells were seeded in 12-well plates for 24 hours. Then they were treated for 24 hours with 10 ng/ml of testosterone. After this step, fresh culture medium containing the sample to be tested was added. The sample was dissolved directly in the culture medium. The concentrations of the sample to be tested were selected based on preliminary cytotoxicity. Untreated cells were used as a negative control, and as a positive control the cells were treated with Serenoa repens at a concentration of 10 $\mu$g/ml. Each sample was tested in duplicate. After 72 hours of exposure, the total RNA was purified from the cells thanks to the reagent RNAlater. After precipitation and centrifugation (30' at 12,000 rpm at 4°C), the RNA was resuspended in 20 $\mu$l of sterile water and its concentration was determined by spectrophotometry. 300 ng of total RNA were transcribed in cDNA using random primers at 37°C for 2 hours in a thermocycler (Applied Byosistems, Foster City, CA).

3.3.3 Analysis of the gene expression profile through Real Time-PCR:

Changes in the gene expression profile are analysed by RT-qPCR using Syber Green as a fluorescent molecule. For $5\alpha$-reductase, two primer sequences were drawn which pair up in the junction region between introns and exons. The uniqueness of pairing and therefore of amplification of the gene occurs thanks to the GENEBANK database. The signal emitted by Syber Green increases in direct proportion with the amount of amplicon produced during the PCR reaction. The detection of the fluorescence emitted in each cycle allows monitoring the PCR reaction during the exponential phase, namely where the first significant increases in the PCR product are related to the initial amount of template.

3.3.4 Result expression

Changes in the gene expression profile are expressed in a relative and not absolute way. All values are initially normalized with respect to a "housekeeping" gene, the actin, which is assumed not to be modulated by the treatment. The analysis starts by differentiating the CT value of the target and the one of the normalizer (DCT).

$$DCT = CT \text{ (target)} - CT \text{ (normalizer)}$$

A normalized CT value is obtained for each sample. Subsequently, data can be expressed as "fold change", namely as a ratio with respect to control (a) or as a percentage with respect to control (b). In any case, it is never a matter of absolute values, but simply of relations with respect to the untreated control.

In case a), the DDCT method is used. For each sample, the value of the normalized DCT is subtracted from the normalized DCT value of the control. This is why it is called DDCT. The values, in logarithmic scale, are then expressed in linear scale according to the following formula:

$$\text{Fold change} = 2 - DDCT$$

In case b), the CT normalized for each single sample (normalized CT) are transformed into fluorescence intensity according to the following formula:

$$\text{Normalized fluorescence intensity} = 2 \wedge -DCTnorm$$

At this point, the percentage of intensity of the sample compared to the control is calculated on a linear scale.

3.4 Method acceptability criteria

For positive control (CP): fold change $\leq 0.5$ or percent inhibition $\geq 50\%$

3.5 Result interpretation

A fold change of $\leq 0.5$ or a reduction rate $\geq 50\%$ compared to the untreated cells indicates a modulation of the investigated gene. This value is compared to the one of the untreated cells to evaluate the efficacy. The statistical analysis is also reported for the evaluation of the significance of the data.

3.6 Test acceptability requirements

|  | Value | Limits | Result |
|---|---|---|---|
| **CP: fold change / percentage decrease** | 87.55 | $\leq 0.5 \geq 50\%$ | Complies |

The test acceptability criteria (see § 2.3) are compliant, so the test is considered valid.

3.7 Results

| HHDPC | 48 h | |
|---|---|---|
|  | **% 5a-reductase (ds) expression** | **% reduction** |
| **5% HMR glycerine solution 1 mg/ml** | 42.87 (16.03) | 57.13 |
| **5% HMR glycerine solution 0.2 mg/ml** | 90.93 (23.48) | 9.07 |
| **Serenoa repens 10 $\mu$g/ml (Positive control)** | 12,45 (2,60) | 87,55 |
| **Negative control** | 100,00 (0,01) | n/a |

| HHDPC | 48 h | |
|---|---|---|
| | % 5α-reductase (ds) expression | % reduction |
| 2% Minoxidil solution 1 mg/ml | 41.96 (22.43) | 58.04 |
| 2% Minoxidil solution 0.5 mg/ml | 89.90 (27.66) | 10.1 |
| Serenoa repens 10 μg/ml (Positive control) | 12.45 (2.60) | 87.55 |
| Negative control | 100.00 (0.01) | n/a |

3.8 Conclusions

The tested sample (1mg/ml of 5% HMR glycerine solution, corresponding to 0.05% HMR) is able to reduce the expression of messenger RNA coding for the enzyme 5-reductase (-57.13%), in cultures of human cells of the dermal papilla, compared to the untreated control, after 48 h of treatment.

EXAMPLES

Some examples of topical formulations containing the carrier composition 7-hydroxymatairesinol (active principle) according to the purposes of the present invention are given for illustrative, but not limitative purposes.

**Shampoo (category: foaming product):**

| | |
|---|---|
| Surfactant blend* | 25-45% |
| Preservative and chelating blend (system) | 0.5% -1.0% |
| Rheological additive (if any) | 0.5-1.0% |
| Carrier composition | 4-8% |
| PH regulator | q.s. at the desired pH |
| Water | q.s. |

*Recommended: Lauroat (*Sodium Lauroyl Oat Amino Acids*)

**Anti-hair fall lotion (Leave on):**

| | |
|---|---|
| Alcohol | 10-30% |
| Preservative and chelating blend (system) | 1.0% -5.0% |
| Carrier composition | 4-8% |
| PH regulator | q.s. at the desired pH |
| Complementary active principles* | 1-3% |
| Water | q.s. |

*Recommended: Trealix (*Trehalose, Hydrolysed Vegetable Protein*)

**Anti-hair fall hydrogel (Leave on):**

| | |
|---|---|
| Alcohol | 10-30% |
| Preservative and chelating blend (system) | 1.0% -5.0% |
| Carrier composition | 4-8% |
| PH regulator | q.s. at the desired pH |
| Rheological additive | 0.5-1.0% |
| Complementary active principles* | 1-3% |
| Water | q.s. |

*Recommended: Trealix (*Trehalose, Hydrolysed Vegetable Protein*)

EP 3 568 131 B1

**Conditioner and cationic mask (rinse off):**

| | |
|---|---|
| Emulsifying blend (system)* | 2.0-6.0% |
| Lipophilic phase (oils, waxes) | 2.0-5.0% |
| Rheological additive | 0.5-1.0% |
| Conditioning blend (system) ** | 4.0-6.0% |
| Carrier composition | 4-8% |
| Preservative and chelating blend (system) | 0.7% -1.5% |
| PH regulator | q.s. at the desired pH |
| Complementary active principles*** | 1-3% |
| Water | q.s. |

*Recommended: Supreme (*Polyglyceryl-3 Rice Branate, Cetearyl Alcohol, Sucrose Stearate*)
**Recommended: Vegequat (*Cocodimonium Hydroxypropyl Hydrolysed Wheat Protein*)
***Xsolve (*Ethyl Ximenynate, Lecithin*) or Leniphenol (*Pinus radiata Bark Extract*)

**Mousse (overnight application):**

| | |
|---|---|
| Surfactant blend* | 7.0-10.0% |
| Alcohol | 10.0-20.0% |
| Carrier composition | 4-8% |
| Preservative and chelating blend (system) | 0.5% -1.0% |
| PH regulator | q.s. at the desired pH |
| Complementary active principles** | 1-3% |
| Water | q.s. |

*Recommended: Undeoat (*Undecylenoyl Oat Amino Acids*)
** Recommended: TREALIX (*Trehalose, Hydrolysed Vegetable Protein*)

It is suggested to add the carrier composition at the end of the formulation, at a temperature of 25°C.

BIBLIOGRAPHIC REFERENCES

[1] Bradford M. (1976). A rapid and sensitive method for the quantition of microgram quantities of protein utilizing the principle of dye-binding. Anal. Biochem. 72, 248-2 54.
[2] Mossman, T. (1993). Rapid colorimetric assay for cellular growth and survival: application to proliferation and cytotoxicity assays. J. Immunol. Methods 65:55-63.

**Claims**

1.  Carrier composition for application on the hair and on the scalp, comprising

    - 7-hydroxymatairesinol (HMR) in concentrations between 2% and 10% by weight on the total weight of the composition,
    - diols and/or triols in a concentration between 30% and 60% by weight on the total weight of the composition,
    - lipoaminoacids in a concentration between 30% and 60% by weight on the total weight of the composition,

    for use in the treatment or prevention of trichological diseases of inflammatory and/or hormonal origin.

2.  Carrier composition for use according to claim 1, wherein said trichological pathologies of inflammatory and/or hormonal origin are pathologies **characterized by** hair loss.

3.  Carrier composition for use according to claim 2, wherein said trichological pathologies of inflammatory and/or

13

hormonal origin, **characterized by** hair loss, are selected from: dandruff, asteatosis, aerated alopecia, seborrhoea, folliculitis, ringworm, tricodynia and androgenetic alopecia.

4. Carrier composition for use according to any one of Claims 1 to 3, wherein diols and/or triols are contained in concentrations ranging from 35% to 50% by weight based on the total weight of the composition.

5. Carrier composition for use according to any one of Claims 1 to 4, wherein lipoaminoacids are contained in concentrations ranging from 45% to 60% by weight based on the total weight of the composition.

6. Carrier composition for use according to any one of Claims 1 to 5, wherein 7-hydroxymatairesinol is contained in concentrations ranging from 3% to 6% by weight based on the total weight of the composition.

7. Carrier composition for use according to any one of Claims 1 to 6, in combination with possible further active principles, suitable eluents and/or excipients, in the form of a formulation.

8. Carrier composition for use according to Claim 7, wherein the formulation comprises said carrier composition in an amount ranging between 4% and 8% by weight based on the total weight of the formulation.

9. Carrier composition for use according to Claim 8, wherein the formulation comprises 7-hydroxymatairesinol as the only active principle.

10. Carrier composition for use according to any one of Claims 7 to 9, wherein the formulation is in the form of liquid, fluid, semi-fluid or consistent formulations.

**Patentansprüche**

1. Trägerzusammensetzung zur Anwendung auf dem Haar und auf der Kopfhaut, umfassend

   - 7-Hydroxymatairesinol (HMR) in Konzentrationen zwischen 2 und 10 Gew.-%, bezogen auf das Gesamtgewicht der Zusammensetzung,
   - Diole und/oder Triole in einer Konzentration zwischen 30 und 60 Gew.-%, bezogen auf das Gesamtgewicht der Zusammensetzung,
   - Lipoaminosäuren in einer Konzentration zwischen 30 und 60 Gew.-%, bezogen auf das Gesamtgewicht der Zusammensetzung,

   zur Verwendung bei der Behandlung oder Vorbeugung von trichologischen Erkrankungen entzündlichen und/oder hormonellen Ursprungs.

2. Trägerzusammensetzung zur Verwendung gemäß Anspruch 1, wobei die trichologischen Pathologien entzündlichen und/oder hormonellen Ursprungs Pathologien sind, die durch Haarausfall gekennzeichnet sind.

3. Trägerzusammensetzung zur Verwendung gemäß Anspruch 2, wobei die trichologischen Pathologien entzündlichen und/oder hormonellen Ursprungs, die durch Haarausfall gekennzeichnet sind, aus Folgenden ausgewählt sind: Schuppen, Asteatose, Alopecia areata, Seborrhoe, Follikulitis, Tinea, Trichodynie und androgenetische Alopezie.

4. Trägerzusammensetzung zur Verwendung gemäß einem der Ansprüche 1 bis 3, wobei Diole und/oder Triole in Konzentrationen im Bereich von 35 bis 50 Gew.-%, bezogen auf das Gesamtgewicht der Zusammensetzung, enthalten sind.

5. Trägerzusammensetzung zur Verwendung gemäß einem der Ansprüche 1 bis 4, wobei Lipoaminosäuren in Konzentrationen im Bereich von 45 bis 60 Gew.-%, bezogen auf das Gesamtgewicht der Zusammensetzung, enthalten sind.

6. Trägerzusammensetzung zur Verwendung gemäß einem der Ansprüche 1 bis 5, wobei 7-Hydroxymatairesinol in Konzentrationen im Bereich von 3 bis 6 Gew.-%, bezogen auf das Gesamtgewicht der Zusammensetzung, enthalten ist.

7. Trägerzusammensetzung zur Verwendung gemäß einem der Ansprüche 1 bis 6, in Kombination mit möglichen weiteren Wirkstoffen, geeigneten Elutionsmitteln und/oder Hilfsstoffen, in Form einer Formulierung.

8. Trägerzusammensetzung zur Verwendung gemäß Anspruch 7, wobei die Formulierung die Trägerzusammensetzung in einer Menge zwischen 4 und 8 Gew.-%, bezogen auf das Gesamtgewicht der Formulierung, umfasst.

9. Trägerzusammensetzung zur Verwendung gemäß Anspruch 8, wobei die Formulierung 7-Hydroxymatairesinol als einzigen Wirkstoff umfasst.

10. Trägerzusammensetzung zur Verwendung gemäß einem der Ansprüche 7 bis 9, wobei die Formulierung in Form von flüssigen, fluiden, semifluiden oder konsistenten Formulierungen vorliegt.

**Revendications**

1. Composition véhicule pour application sur les cheveux et sur le cuir chevelu, comprenant

   - du 7-hydroxymatairésinol (HMR) à des concentrations comprises entre 2 % et 10 % en poids sur le poids total de la composition,
   - des diols et/ou des triols à une concentration comprise entre 30 % et 60 % en poids sur le poids total de la composition,
   - des lipoaminoacides à une concentration comprise entre 30 % et 60 % en poids sur le poids total de la composition,

   pour une utilisation dans le traitement ou la prévention de maladies trichologiques d'origine inflammatoire et/ou hormonale.

2. Composition véhicule pour une utilisation selon la revendication 1, dans laquelle lesdites pathologies trichologiques d'origine inflammatoire et/ou hormonale sont des pathologies **caractérisées par** une perte de cheveux.

3. Composition véhicule pour une utilisation selon la revendication 2, dans laquelle lesdites pathologies trichologiques d'origine inflammatoire et/ou hormonale, **caractérisées par** une perte de cheveux, sont sélectionnées parmi : les pellicules, l'astéatose, l'alopécie aérée, la séborrhée, la folliculite, la teigne tondante, la tricodynie et l'alopécie androgénétique.

4. Composition véhicule pour une utilisation selon l'une quelconque des revendications 1 à 3, dans laquelle les diols et/ou les triols sont contenus à des concentrations allant de 35 % à 50 % en poids sur la base du poids total de la composition.

5. Composition véhicule pour une utilisation selon l'une quelconque des revendications 1 à 4, dans laquelle les lipoaminoacides sont contenus à des concentrations allant de 45 % à 60 % en poids sur la base du poids total de la composition.

6. Composition véhicule pour une utilisation selon l'une quelconque des revendications 1 à 5, dans laquelle le 7-hydroxymatairésinol est contenu à des concentrations allant de 3 % à 6 % en poids sur la base du poids total de la composition.

7. Composition véhicule pour une utilisation selon l'une quelconque des revendications 1 à 6, en combinaison avec des principes actifs supplémentaires possibles, des éluants et/ou des excipients appropriés, sous la forme d'une formulation.

8. Composition véhicule pour une utilisation selon la revendication 7, dans laquelle la formulation comprend ladite composition véhicule dans une quantité allant de 4 % à 8% en poids rapporté au poids total de la formulation.

9. Composition véhicule pour une utilisation selon la revendication 8, dans laquelle ladite formulation comprend du 7-hydroxymatairésinol comme seul principe actif.

10. Composition véhicule pour une utilisation selon l'une quelconque des revendications 7 à 9, dans laquelle la formulation est sous la forme de formulations liquides, fluides, semi-fluides ou constantes.

Figure 1

Figure 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1513514 A
- WO 2015173345 A

**Non-patent literature cited in the description**

- **BRADFORD M.** A rapid and sensitive method for the quantition of microgram quantities of protein utilizing the principle of dye-binding. *Anal. Biochem.,* 1976, vol. 72, 248-2, 54
- **MOSSMAN, T.** Rapid colorimetric assay for cellular growth and survival: application to proliferation and cytotoxicity assays. *J. Immunol. Methods,* 1993, vol. 65, 55-63